# EUROPEAN PATENT SPECIFICATION

(11) **EP 2 659 919 B1**
(45) Date of publication and mention of the grant of the patent: **10.12.2014**
(21) Application number: 12166674.7
(22) Date of filing: 03.05.2012
(51) Int. Cl.: A61M 1/16

(54) **Method for safety relevant parameter setting in a medical apparatus and medical apparatus**
Verfahren zur sicherheitsrelevanten Parametereinstellung in einer medizinischen Vorrichtung und medizinische Vorrichtung
Procédé de réglage de paramètres pertinents de sécurité dans un appareil médical et appareil médical

(43) Date of publication of application: 06.11.2013
(73) Proprietor: B. Braun Avitum AG, 34212 Melsungen (DE)
(72) Inventor: Dolgos, Sándor, Dr., 2000 Szentendre (HU); Schin, Róbert D., 1029 Budapest (HU); Szamkó, Péter, 2131 Göd (HU); Bogatin, György, 1116 Budapest (HU); Kinoranyi, Gabor, 1045 Budapest (HU)
(74) Representative: Winter, Brandl, Fürniss, Hübner, Röss, Kaiser, Polte - Partnerschaft mbB

(56) References cited:
- WO-A1-96/41292
- WO-A1-2006/131775
- US-A- 4 990 258
- US-A1- 2002 179 505
- US-A1- 2005 256 444

## Description

The invention relates to a method for setting at least one safety relevant parameter of a microprocessor controlled medical apparatus, whereby processes of the medical apparatus are operated based at least on said parameter setting, and the medical apparatus comprises means for manual selection and amendment of the parameter setting by an operator.

The invention further relates to a medical apparatus using this method.

Modern medical apparatuses are often controlled by a microprocessor that, for example, operates pumps, reads sensors and communicates with an operator via a user interface like a monitor, keypad and/or touchscreen. This user interface can make use of text, pictograms and/or graphical icons to guide an operator through the setup and give him/her necessary information during a therapy that is performed by the medical apparatus.

Thereby, parameter input is an essential part of such medical equipment. For example, extracorporal blood treatment (ECB) involves the continuous withdrawal of blood from a patient, where the blood is processed within a medical device outside of the patient and is then returned to the patient. Parameters like the ultrafiltration volume, the therapy time and the ultrafiltration rate can be input by a nurse depending on the patient's prescription, and the medical apparatus can then individually perform the therapy for each patient. The traditional way is the separate input of parameters, i.e. one by one entering the values by any means, and confirming these parameters before they are implemented. However, some of these parameters are mutually dependent from each other and possibly from the time passed during the therapy, which can cause usability problems. Therefore, U.S. Patent Application US 2005/0256444 A1, for example, suggests a user interface for a blood treatment device which allows to select and review a series of parameter settings and to implement the settings in batch manner.

In addition, the functional safety of medical equipment has been very much developed and essentially improved in the past two decades. In particular, if a patient is constantly connected to a medical apparatus like an extracorporal blood treatment device, any failure in entering or implementing a proper prescription can have negative impacts on the therapy and the patient's health. Therefore, it has to be guaranteed that the processes of a medical apparatus are actually controlled by the parameters that have been input by an operator.

International Patent Application WO 2006/131775 A1, for example, describes a medical apparatus comprising a user interface for setting parameters that includes a screen for visualizing values of said parameters, a main control unit connected to the interface, a first memory and a video memory both connected to the main control unit for storing data corresponding to images on the screen. The main control unit allows setting of a new value for a parameter, displays the new value on the screen region, stores the new value in the first memory and captures from the video memory data that is representative of said screen region. Hereby, it can be verified from said representative data whether the displayed value corresponds to the value in the first memory, which is used for the processes of the medical apparatus during therapy.

Furthermore, the processes of a medical apparatus like an extracorporal blood treatment device are often controlled by a control software unit and additionally monitored by a supervisor software unit. Both software units have to be provided with the same parameters and statuses via two channels, and it can be considered an essential part of the functional safety of the medical apparatus to guarantee that the supervisor software's parameter and/or status is equal to the one(s) confirmed by the user and to those one(s) being used by the functional control unit. For this purpose, the parameters of the two channels can be displayed to the nurse on a screen, for example, who then has to compare the values for each software unit and can take measures, if they differ from each other. However, this requires awareness, time and concentration, whereby human decisions always involve certain risks.

It is therefore an objective of the invention to provide an improved method for safety relevant input of parameters with little handling need and good handling consistency. It is another objective of the invention to provide a corresponding medical apparatus.

According to the invention, this objective is achieved by a method having the features of independent Claim 1. Advantageous refinements of this method are set forth in dependent Claims 2 through 12. The objective is also achieved by a medical apparatus according to Claim 13.

The method according to the invention can be used for setting at least one safety relevant parameter of a microprocessor controlled medical apparatus, whereby processes of the medical apparatus are operated based at least on said parameter setting. Thereby, the medical apparatus comprises a software component driving a user interface connected to a screen and means for manual selection and amendment of the parameter settings by an operator. The method comprises at least the following steps:
- displaying by means of the user interface the at least one safety relevant parameter in an input window on the screen;
- adjusting said parameter in the input window upon manual selection by an operator, and representing the new parameter in a screen image by means of a video RAM;
- communicating data regarding the new parameter from the user interface to a control unit and to a supervisor unit of the medical apparatus after a parameter setting acceptance operation is actuated by the operator;
- capturing the screen image from the video RAM and decoding the screen image to data regarding the new parameter;
- communicating the decoded data regarding the new parameter to the supervisor unit;
- comparing the data regarding the new parameter communicated from the user interface with the data regarding the new parameter communicated from the video RAM, wherein the result of the comparison leads to:
   - generating a signal, if the data regarding the new parameter communicated from the user interface differs from the data regarding the new parameter communicated from the video RAM, or if data regarding the new parameter communicated from the video RAM did not arrive within a time-out at the supervisor unit after the parameter setting acceptance operation; or
   - controlling processes of the medical apparatus by means of the control unit at least based on the data regarding the new parameter received by the control unit and communicating data regarding the running processes to the supervisor unit, if the data regarding the new parameter communicated from the user interface does not differ from the data regarding the new parameter communicated from the video RAM, and checking the processes by means of the supervisor unit.

This method guarantees that the processes of the medical apparatus are performed based on those parameter settings that have been set and confirmed by an operator. If the communication of data regarding a new parameter setting from the user interface to the control unit and supervisor unit is somehow faulty, the video RAM will capture the parameter setting that has really been set by the operator and will communicate this data via a different channel. Both data regarding a new parameter setting can then be compared to generate a signal, if an inconsistency is detected. This signal can be used to issue an alarm, for example, which can be a visual and/or acoustic alarm that informs the operator of a problem with parameter settings. Thus, an operator like a nurse does not have to compare parameters on a screen, for example, because this is done automatically. The method according to the invention requires very little handling need and, consequently, less concentration and time of the user. However, the user is still to compare the settings of the machine to the prescription of the responsible physician.

A user also does not have to know whether a parameter setting is safety relevant or not. This improves the handling consistency, because safety relevant parameters do not have to be confirmed by pressing an additional hardware button, for example, whereas non-safety relevant parameters might not have to be confirmed this way. With the invention, the user will only have to set the parameters and the medical apparatus will take care of the parameters depending on their safety relevance. Thereby, safety relevant parameters are displayed in a safety relevant input window and represented in the video RAM and non-safety relevant parameters will not, for example.

The usability of the medical apparatus is improved by these measures, and the product costs for the equipment are lower than for other known solutions without reducing the level of functional safety that has already been achieved by these known solutions. The manufacturer needs equipment, which is cheaper to produce and cheaper to develop and maintain. Thus, the complexity of an equipment hardware-software system is also a cost issue, since less complexity means less
engineering effort in development and maintenance. Preferably, the screen according to the invention is a single channel display, and similar software can be used for the display and the screen capture feature, which keeps the complexity low.

The solution according to International Patent Application WO 2006/131775 A1, for example, is very complex, since it needs periodical comparisons in three relations of four memory sets, whereas the invention needs comparisons in only one relation of two memory sets. Furthermore, the solution according to said international application provides for safety relevant parameters only and not for status, but in a preferred embodiment of the invention, a parameter comprises data text, value, unit and/or the operational status of the medical apparatus. Therefore, a parameter within the meaning of this invention is not only a value of an ultrafiltration volume, for example, but might also be a status.

The operational status of the medical apparatus can be a therapy mode in which the medical apparatus performs a therapy on a patient, or a preparation mode in which the therapy is prepared, for example. Other statuses can also be considered, and at least the supervisor unit operates depending on the current status of the medical apparatus. Thus, the safety of monitoring the processes can be improved depending on the current status of the medical apparatus, and the solution according to said international application WO 2006/131775 A1 has weaker functional safety than the invention. Furthermore, more than one parameter can be set and handled in batch manner by the method according to the invention. This further increases the usability of the device.

According to one (separate) aspect of the invention, data regarding the parameter communicated from the user interface to the control unit may be cyclically compared to the data regarding the parameter communicated from the user interface to the supervisor unit, and a signal is generated, if these data differ from each other.

Preferably, a signal can inhibit the control of the processes based on new parameter settings so that the processes do not start or are based on current parameter settings.

This ensures that processes are not controlled based on inconsistent parameter settings as long as they are not confirmed.

The parameter setting acceptance operation can be actuated by pressing a soft button on the screen or by pressing a hardware button. Furthermore, another confirmation window can be provided to confirm an acceptance operation.

The invention further comprises a medical apparatus, comprising at least a software component driving a user interface connected to a screen and means for manual selection and amendment of parameter settings by an operator, whereby processes of the medical apparatus are operated based on at least these parameter settings, and the medical apparatus comprises means configured to perform the method described above for setting safety relevant parameters. In one embodiment of the invention, the medical apparatus is an extracorporal blood treatment device that performs hemodialysis, hemofiltration, hemodialfiltration, ultrafiltration and/or plasmapheresis, for example, but the invention is not limited to this application. Since safety relevant parameter setting can be important for all kinds of medical apparatuses, the invention can have advantages for other applications, too.

Additional advantages, special features and practical refinements of the invention can be gleaned from the dependent claims and from the presentation below of preferred embodiments making reference to the figures.

The figures show the following:
- Fig. 1: an example of a system architecture for implementing the method according to the invention; and
- Fig. 2: a schematic illustration of CPUs and video RAM in connection to a screen.

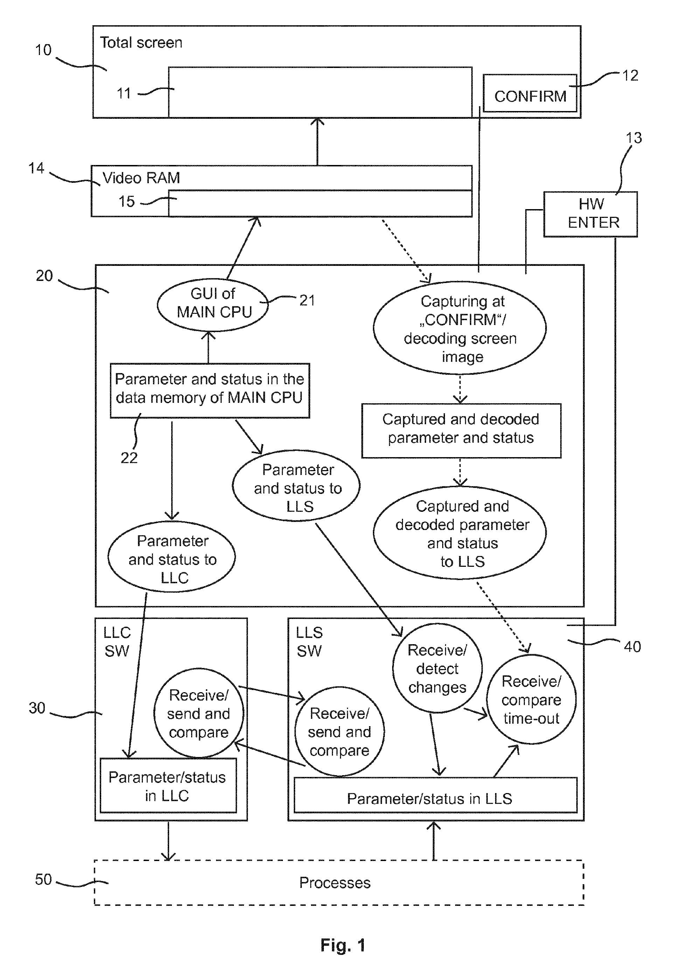

Fig. 1 shows a diagram depicting the basic features of a system architecture of a microprocessor controlled extracorporal blood treatment device (ECB device), which is one example of a medical apparatus for which the invention can be used. The ECB device comprises at least an extracorporal blood circuit, pumps and sensors (not shown) and a software component 20 that controls processes 50 for performing the therapy functions of the ECB device. The processes can be performed on parameters like the ultrafiltration volume, the ultrafiltration rate and/or the therapy time, for example, which are set for each patient individually depending on the patient's description.

The ECB device also comprises a screen 10 on which a parameter input window 11 can be displayed by the graphical user interface 21 (GUI) of the software component 20 that runs on the MAIN CPU of the microprocessor controlled ECB device. Preferably, the screen 10 is a touchscreen which allows displaying and inputting information to/by a user of the ECB device.

According to the embodiment of Fig. 1, the software component 20 provides current parameter settings including statuses to two software units 30 and 40. These current parameter settings and statuses have been input by a user via the parameter input window 11. The first software unit 30 is a control unit (LLC) that controls the processes 50 of the device based on these parameter settings and statuses. Thereby, the control software 30 drives pumps, feed-back controls, dialysate composition, sets valve positions, etc. The same parameter settings and statuses are provided to the supervisor unit (LLS) 40 that also receives data from the processes 50 and monitors these processes based on said received data and the parameter settings/statuses. It checks the pump speed, dialysate composition, valve positions, etc. using the same values as the control unit 30, which were confirmed by the user. This can be called a two channel architecture and is illustrated on the left side of the system architecture in Fig. 1 with continuous arrows.

The software component 20 drives the user interface 21 that is connected to the screen 10 and comprises means for displaying the parameter input window 11 on the screen 10. Data text, value and unit and/or operational status of the MAIN CPU are displayed on the single channel display 10. When parameters have been input in this input window 11 by an operator, they can be confirmed by touching a software "CONFIRM" button 12, for example. Alternatively or in addition, a hardware button 13 can be provided which can be pressed to confirm an amended setting. Parameter and status can be entered without confirmation, or changed data can be confirmed automatically. Upon confirmation, the software component 20 stores the parameter settings and status in the local memory of the input window 11 or the data memory 22 of the MAIN CPU, respectively. The corresponding input window 11 can be closed, and the processes 50 of the ECB device are controlled by the control unit 30 based on these amended/new parameter settings. As mentioned before, the amended/new parameter settings are also communicated to the supervisor unit 40. Based on these settings and data received from the processes 50, the supervisor unit 40 can check whether the processes 50 run correctly.

Furthermore, there is a data connection between the control unit 30 and the supervisor software 40 in order to exchange parameters and status. Thereby, data in their slave CPUs are cyclically received/sent and then compared to be equal except for limited duration transients. If the parameters and statuses used by the control unit 30 and the supervisor software 40 are not equal over transient time, an alarm is issued. If this difference is detected by the control unit 30, a control alarm is issued, whereas as supervisor alarm is issued, if the supervisor unit 40 detects the difference.

At the same time, a video RAM 14 is operated between the screen 10 and the GUI 21 of the MAIN CPU, which comprises a special video RAM 15 for parameter input window 11. The parameter input window 11 serves for setting safety relevant parameters. Therefore, this input window 11 can also be called a safety relevant window, and safety relevant parameters can be captured by means of the video RAM 15 in order to provide them to the supervisor unit 40 via a parallel channel. This additional channel from the video RAM 15 to the supervisor unit 40 only is illustrated with dashed arrows on the right side of the system architecture in Fig. 1.

Thereby, data text, value and unit and/or operational status of the MAIN CPU is represented in a screen image by means of the video RAM 15, and this screen image is captured/read back by a TASK in the MAIN CPU when the soft "CONFIRM" button 12 is pressed on the screen 10, for example. The screen image is then decoded to data text, value and unit and/or operational status by another TASK in the MAIN CPU. Any method can be used for this decoding, e.g. comparing it to a desired image, or any pattern recognition method. The decoded data is then sent to the supervisor unit 40 as described before. Thus, the supervisor unit 40 receives parameter and status data via the user interface 21 of the MAIN CPU and from the video RAM 15.

The data received from the user interface 21 is sent to a SECOND SLAVE CPU, where any change is detected to start-time of receiving decoded screen. The decoded screen is communicated from the MAIN CPU to SECOND SLAVE CPU and then compared to the data in the SECOND SLAVE CPU by a TASK in the SECOND SLAVE CPU. If parameter and status received by the supervisor unit 40 via the two channels differ from each other or a time-out is expired, a supervisor alarm is issued. Thus, an error in software can occur, if wrong contents were detected on the screen, or if the detection did not happen within a time limit.

Thereby, in safety relevant input the input of parameters and statuses are equally important. The status expresses whether the medical apparatus and the corresponding software are in preparation mode or therapy mode, for example. Having just safety relevant input for parameters, but not for statuses would be an unsafe design of the software system. Any software unit, therefore the supervisor unit 40, too, contains features, which are operated depending on the status / statuses of the software. The status transition is normally triggered by pressing a button and then confirming a confirmation window ("Are you sure..."), i.e. by pressing another button once more. By this trigger the software system is supposed to change its status, i.e. all the components are supposed to change their statuses in the same way. If the design cannot guarantee that the supervisor unit 40 is in the status, which used to be confirmed by the user, then the design remains unsafe regardless of parameters being input in a safe way. Thereby, if a user changes the status of the medical apparatus via the user interface 21, it has to be guaranteed that this change of status is safely accounted for.

For example, if the safety relevant input of the set ultrafiltration volume (UF volume) is part of the design of the software system, one can be sure that the supervisor software memory will contain the correct set UF volume value that has been confirmed by the user. Nevertheless, the supervisor unit 40 takes care of the UF volume (measures, calculates and compares it to the set value) in "Therapy" main phase/status only. It does not take care of the UF volume e.g. in "Preparation" main phase/status. Consequently, if the design cannot guarantee that the supervisor unit 40 is in the "Therapy" status, which used to be confirmed by the user and not in the "Preparation" status any more, then the safely input set UF volume will be neglected, i.e. the UF volume will not be taken care of.

Furthermore, parameters and/or status do not have to be adjusted one by one, but they can be set in batch manner, too. For example, several settings can manually be performed by the user in the input window 11, and they are handled in the same way in parallel upon confirmation by the operator.

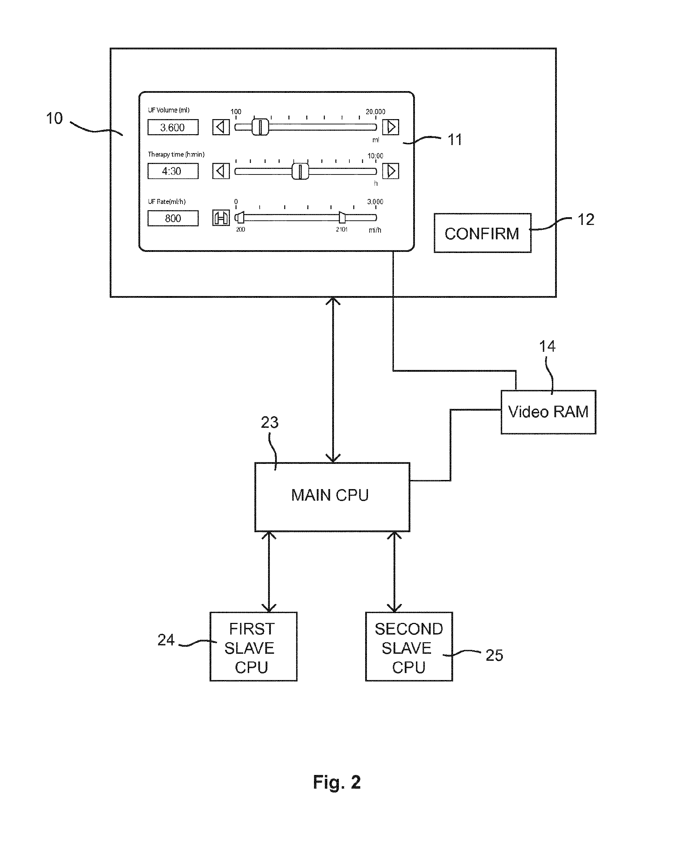

The invention is further described by means of Fig. 2, which shows a schematic illustration of CPUs and video RAM in connection to a screen. The input window 11 is displayed on the screen 10, which can be a single channel display. Thereby, the input window 11 is displayed by means of the graphical user interface of the MAIN CPU 23 so that data text, value, unit and/or operational status of the MAIN CPU (hardware of software component 20) are displayed. This MAIN CPU 23 is in connection to a FIRST SLAVE CPU 24 and SECOND SLAVE CPU 25. The FIRST SLAVE CPU 24 is associated to the control unit 30, whereas the SECOND SLAVE CPU 25 is associated to the supervisor unit 40.

Upon confirmation by the soft button 12, for example, data text, value, unit of at least one adjusted parameter setting and/or operational status of the MAIN CPU 23 is represented in a screen image and captured (read back) by a video RAM 14 by a TASK in the MAIN CPU 23. The captured screen image is then decoded to data text, value, unit and/or operational status by a TASK in the MAIN CPU 23. Captured and decoded data is communicated from MAIN CPU 23 to SECOND SLAVE CPU 25 (hardware of supervisor unit 40) and then compared to the data in the SECOND SLAVE CPU 25 by a TASK in the SECOND SLAVE CPU 25 (data/task in hardware of supervisor unit 40). If any change is detected in the SECOND SLAVE CPU 25, a signal is triggered. Furthermore, data in both SLAVE CPUs 24 and 25 are cyclically received and sent and then compared to be equal except limited duration transients.

### List of Reference Numerals

- 10: Screen, touchscreen, display
- 11: Parameter input window, safety relevant input window
- 12: Soft "CONFIRM" button
- 13: Hardware enter button
- 14: Video RAM
- 15: Video RAM for safety relevant window
- 20: Software component
- 21: Graphical user interface, GUI
- 22: Storage unit, data memory of MAIN CPU
- 23: MAIN CPU
- 24,25: SLAVE CPU
- 30: Control unit, LLC
- 40: Supervisor unit, LLS
- 50: Processes

## Claims

1. Method for setting at least one safety relevant parameter of a microprocessor controlled medical apparatus, whereby processes (50) of the medical apparatus are operated based at least on said parameter setting and the medical apparatus comprises a software component (20) driving a user interface (21) connected to a screen (10), and the medical apparatus further comprises means for manual selection and amendment of the parameter setting by an operator, the method comprising:
- displaying by means of the user interface (21) the at least one safety relevant parameter in an input window (11) on the screen (10);
- adjusting said parameter in the input window (11) upon manual selection by an operator, and representing the new parameter in a screen image by means of a video RAM (14);
- communicating data regarding the new parameter from the user interface (21) to a control unit (30) and to a supervisor unit (40) of the medical apparatus after a parameter setting acceptance operation is actuated by the operator;
- capturing the screen image from the video RAM (14) and decoding the screen image to data regarding the new parameter;
- communicating the decoded data regarding the new parameter to the supervisor unit (40);
- comparing the data regarding the new parameter communicated from the user interface (21) with the data regarding the new parameter communicated from the video RAM (14), wherein the result of the comparison leads to:
- generating a signal, if the data regarding the new parameter communicated from the user interface (21) differs from the data regarding the new parameter communicated from the video RAM (14), or if data regarding the new parameter communicated from the video RAM (14) did not arrive within a time-out at the supervisor unit (40) after the parameter setting acceptance operation; or
- controlling processes (50) of the medical apparatus by means of the control unit (30) at least based on the data regarding the new parameter received by the control unit (30) and communicating data regarding the running processes (50) to the supervisor unit (40), if the data regarding the new parameter communicated from the user interface (21) does not differ from the data regarding the new parameter communicated from the video RAM (14), and checking the processes (50) by means of the supervisor unit (40).

2. Method according to Claim 1, wherein the screen (10) is a single channel display.

3. Method according to one or both of Claims 1 and 2, wherein the parameter comprises data text, value, unit and/or the operational status of the medical apparatus.

4. Method according to one or more of Claims 1 to 3, wherein the operational status of the medical apparatus can at least be a therapy mode in which the medical apparatus performs a therapy on a patient, or a preparation mode in which the therapy is prepared.

5. Method according to Claim 4, wherein at least the supervisor unit (40) operates depending on the operational status of the medical apparatus.

6. Method according to one or more of Claims 1 to 5, wherein more than one parameter is set and handled in batch manner.

7. Method according to one or more of Claims 1 to 6, wherein non-safety relevant parameters are not represented in the video RAM (14).

8. Method according to one or more of Claims 1 to 7, wherein the data regarding the parameter communicated from the user interface (21) to the control unit (30) is cyclically compared to the data regarding the parameter communicated from the user interface (21) to the supervisor unit (40), and a signal is generated, if these data differ from each other.

9. Method according to one or more of Claims 1 to 8, wherein the signal issues an alarm.

10. Method according to Claim 9, wherein the alarm is a visual and/or acoustic alarm.

11. Method according to one or more of Claims 1 to 10, wherein the signal inhibits the control of the processes (50) based on new parameter settings so that the processes (50) do not start or are based on current parameter settings.

12. Method according to one or more of Claims 1 to 11, wherein the parameter setting acceptance operation is actuated by pressing a soft button (12) on the screen (10) or by pressing a hardware button (13).

13. Medical apparatus, comprising at least a software component (20) driving a user interface (21) connected to a screen (10) and means for manual selection and amendment of parameter settings by an operator, whereby processes (50) of the medical apparatus are operated based on at least these parameter settings, **characterized in that** the medical apparatus comprises means configured to perform the method according to one or more of Claims 1 to 12 for setting safety relevant parameters.

14. Medical apparatus according to Claim 13, wherein the medical apparatus is an extracorporal blood treatment device.

## Patentansprüche

1. Verfahren zum Einstellen mindestens eines sicherheitsrelevanten Parameters einer mikroprozessorgesteuerten medizinischen Vorrichtung, wodurch Prozesse (50) der medizinischen Vorrichtung basierend auf dem wenigstens einen Parameter betrieben werden und die medizinische Vorrichtung eine Softwarekomponente (20) umfasst, die eine Benutzerschnittstelle (21) ansteuert, die mit einem Bildschirm (10) verbunden ist, und die medizinische Vorrichtung ferner Mittel zur manuellen Auswahl und Änderung der Parametereinstellung durch einen Benutzer umfasst, wobei das Verfahren umfasst:
- Anzeigen des wenigstens einen sicherheitsrelevanten Parameters in einem Eingabefenster (11) auf dem Bildschirm (10) durch Mittel der Benutzerschnittstelle (21);
- Einstellen des Parameters im Eingabefenster (11) auf eine manuelle Auswahl eines Benutzers hin, und Wiedergeben des neuen Parameters in einem Bildschirmbild durch Mittel eines Video-RAMs (14);
- Übertragen der den neuen Parameter betreffenden Daten von der Benutzerschnittstelle an eine Steuereinheit (30) und eine Überwachungseinheit (40) der medizinischen Vorrichtung, nachdem eine Parametereinstellungs-Annahmebedienung von einem Benutzer ausgeführt wird;
- Erfassen des Bildschirmbildes von dem Video-RAM (14) und Dekodieren des Bildschirmbildes in die den neuen Parameter betreffenden Daten;
- Übertragen der dekodierten, den neuen Parameter betreffenden Daten an die Überwachungseinheit (40);
- Vergleichen der den neuen Parameter betreffenden Daten von der Benutzerschnittstelle mit den den neuen Parameter betreffenden Daten von dem Video-RAM (14), wobei das Ergebnis des Vergleichs dazu führt:
- Erzeugen eines Signals, wenn die den neuen Parameter betreffenden Daten von der Benutzerschnittstelle von den den neuen Parameter betreffenden Daten abweichen, die von dem Video-RAM (14) übertragen werden, oder, wenn die den neuen Parameter betreffenden Daten nicht innerhalb eines Time-outs nach der Parameterannahmebetätigung bei der Überwachungseinheit (40) angekommen sind; oder
- Steuern der Prozesse (50) der medizinischen Vorrichtung durch Mittel der Steuereinheit (30) wenigstens basierend auf den Daten, die den neuen Parameter betreffen und die von der Steuereinheit (30) empfangen werden, und Übertragen der Daten, die die laufenden Prozesse (50) betreffen, an die Überwachungseinheit (40), wenn die den neuen Parameter betreffenden Daten von der Benutzerschnittstelle (21), von den den neuen Parameter betreffenden Daten des Video-RAMs (14) nicht abweichen, und Überprüfen der Prozesse (50) durch Mittel der Überwachungseinheit (40).

2. Verfahren nach Anspruch 1, wobei der Bildschirm (10) eine Einzelkanalanzeige ist.

3. Verfahren nach einem oder beiden Ansprüchen 1 und 2, wobei der Parameter Datentext, Wert, Einheit und/oder den Betriebsstatus der medizinischen Vorrichtung umfasst.

4. Verfahren nach einem oder mehreren der Ansprüche 1 bis 3, wobei der Betriebsstatus der medizinischen Vorrichtung wenigstens ein Therapiemodus, in dem die medizinische Vorrichtung eine Therapie an einem Patienten durchführt, oder ein Vorbereitungsmodus sein kann, in dem die Therapie vorbereitet wird.

5. Verfahren nach Anspruch 4, wobei wenigstens die Überwachungseinheit (40) in Abhängigkeit von dem Betriebsstatus der medizinischen Vorrichtung arbeitet.

6. Verfahren nach einen oder mehreren der Ansprüche 1 bis 5, wobei mehr als ein Parameter auf Batch-Weise eingestellt und verarbeitet wird.

7. Verfahren nach einem oder mehreren Ansprüchen 1 bis 6, wobei nichtsicherheitsrelevante Parameter nicht in dem Video-RAM (14) wiedergegeben werden.

8. Verfahren nach einem oder mehreren der Ansprüche 1 bis 7, wobei die den Parameter betreffenden Daten, die von der Benutzerschnittstelle (21) an die Steuereinheit (30) übertragen werden, zyklisch mit den den Parameter betreffenden Daten verglichen werden, die von der Benutzerschnittstelle (21) an die Überwachungseinheit (40) übertragen werden, und ein Signal erzeugt wird, wenn diese Daten voneinander abweichen.

9. Verfahren nach einem oder mehreren der Ansprüche 1 bis 8, wobei das Signal einen Alarm ausgibt.

10. Verfahren nach Anspruch 9, wobei der Alarm ein visueller und/oder akustischer Alarm ist.

11. Verfahren nach einem oder mehreren der Ansprüche 1 bis 10, wobei das Signal das Steuern der Prozesse (50) sperrt, die auf dem neuen Parameter basieren, so dass die Prozesse (50) nicht starten oder auf den aktuellen Parametereinstellungen basieren.

12. Verfahren nach einem oder mehreren der Ansprüche 1 bis 11, wobei die Parametereinstellungs-Annahmebedienung durch Drücken einer Softtaste (12) auf dem Bildschirm (10) oder durch Drücken einer Hardwaretaste (13) betätigt wird.

13. Medizinische Vorrichtung, die wenigstens eine Softwarekomponente (20), die eine Benutzerschnittstelle (21) betreibt, die mit einem Bildschirm (10) verbunden ist, und Mittel zur manuellen Auswahl und Änderung einer Parametereinstellung durch einen Benutzer umfasst, wodurch Prozesse (50) der medizinischen Vorrichtung basierend auf dem wenigstens diesen Parametereinstellungen betrieben werden, **dadurch gekennzeichnet, dass** die medizinische Vorrichtung Mittel umfasst, die dazu eingerichtet sind, das Verfahren zum Einstellen sicherheitsrelevanter Parameter nach einem oder mehreren der Ansprüche 1 bis 12 auszuführen.

14. Medizinische Vorrichtung nach Anspruch 13, wobei die medizinische Vorrichtung ein Gerät zur extrakorporalen Blutbehandlung ist.

## Revendications

1. Procédé de réglage d'au moins un paramètre relatif à la sécurité d'un appareil médical commandé par un microprocesseur, moyennant quoi des processus (50) de l'appareil médical sont exploités sur la base d'au moins ledit réglage de paramètre, et l'appareil médical comprend un composant logiciel (20) commandant une interface d'utilisateur (21) connectée à un écran (10), et l'appareil médical comprend en outre un moyen de sélection manuelle et de modification du réglage de paramètre par un opérateur, le procédé comprenant les étapes ci-dessous consistant à :
- afficher, au moyen de l'interface d'utilisateur (21), ledit au moins un paramètre relatif à la sécurité dans une fenêtre d'entrée (11) sur l'écran (10) ;
- ajuster ledit paramètre dans la fenêtre d'entrée (11) suite à une sélection manuelle exécutée par un opérateur, et représenter le nouveau paramètre dans une image d'écran au moyen d'une mémoire RAM vidéo (14) ;
- communiquer des données relatives au nouveau paramètre de l'interface d'utilisateur (21) à une unité de commande (30) et à une unité de supervision (40) de l'appareil médical, après qu'une opération d'acceptation de réglage de paramètre a été mise en oeuvre par l'opérateur ;
- capturer l'image d'écran à partir de la mémoire RAM vidéo (14) et décoder l'image d'écran en des données relatives au nouveau paramètre ;
- communiquer les données décodées relatives au nouveau paramètre à l'unité de supervision (40) ;
- comparer les données relatives au nouveau paramètre communiquées à partir de l'interface d'utilisateur (21) aux données relatives au nouveau paramètre communiquées à partir de la mémoire RAM vidéo (14), dans lequel le résultat de la comparaison aboutit à :
- générer un signal, si les données relatives au nouveau paramètre communiquées à partir de l'interface d'utilisateur (21) diffèrent des données relatives au nouveau paramètre communiquées à partir de la mémoire RAM vidéo (14), ou si les données relatives au nouveau paramètre communiquées à partir de la mémoire RAM vidéo (14) ne sont pas arrivées au cours d'une période de temporisation, au niveau de l'unité de supervision (40), après l'opération d'acceptation de réglage de paramètre ; ou
- commander des processus (50) de l'appareil médical au moyen de l'unité de commande (30), au moins sur la base des données relatives au nouveau paramètre reçues par l'unité de commande (30), et communiquer des données relatives aux processus en cours (50) à l'unité de supervision (40), si les données relatives au nouveau paramètre communiquées à partir de l'interface d'utilisateur (21) ne diffèrent pas des données relatives au nouveau paramètre communiquées à partir de la mémoire RAM vidéo (14), et vérifier les processus (50) au moyen de l'unité de supervision (40).

2. Procédé selon la revendication 1, dans lequel l'écran (10) correspond à un affichage de canal unique.

3. Procédé selon l'une ou l'autre des revendications 1 et 2, dans lequel le paramètre comprend une unité, une valeur, un texte de données, et/ou l'état opérationnel de l'appareil médical.

4. Procédé selon l'une quelconque ou plusieurs des revendications 1 à 3, dans lequel l'état opérationnel de l'appareil médical peut au moins être un mode de traitement thérapeutique dans lequel l'appareil médical met en oeuvre un traitement thérapeutique sur un patient, ou un mode de préparation dans lequel le traitement thérapeutique est préparé.

5. Procédé selon la revendication 4, dans lequel au moins l'unité de supervision (40) fonctionne selon l'état opérationnel de l'appareil médical.

6. Procédé selon l'une quelconque ou plusieurs des revendications 1 à 5, dans lequel plus d'un paramètre est réglé et traité de façon discontinue.

7. Procédé selon l'une quelconque ou plusieurs des revendications 1 à 6, dans lequel des paramètres non relatifs à la sécurité ne sont pas représentés dans la mémoire RAM vidéo (14).

8. Procédé selon l'une quelconque ou plusieurs des revendications 1 à 7, dans lequel les données relatives au paramètre communiquées de l'interface d'utilisateur (21) à l'unité de commande (30) sont comparées de manière cyclique aux données relatives au paramètre communiquées à partir de l'interface d'utilisateur (21) à l'unité de supervision (40), et un signal est généré, si ces données sont différentes.

9. Procédé selon l'une quelconque ou plusieurs des revendications 1 à 8, dans lequel le signal émet une alarme.

10. Procédé selon la revendication 9, dans lequel l'alarme est une alarme visuelle et/ou une alarme acoustique.

11. Procédé selon l'une quelconque ou plusieurs des revendications 1 à 10, dans lequel le signal inhibe la commande des processus (50) sur la base de nouveaux réglages de paramètres, de sorte que les processus (50) ne démarrent pas, ou de sorte qu'ils sont basés sur des réglages de paramètres en cours.

12. Procédé selon l'une quelconque ou plusieurs des revendications 1 à 11, dans lequel l'opération d'acceptation de réglage de paramètre est initiée en appuyant sur un bouton logiciel (12) sur l'écran (10) ou en appuyant sur un bouton matériel (13).

13. Appareil médical, comprenant au moins un composant logiciel (20) commandant une interface d'utilisateur (21) connectée à un écran (10) et un moyen de sélection manuelle et de modification de réglages de paramètres par un opérateur, moyennant quoi des processus (50) de l'appareil médical sont exploités sur la base d'au moins ces réglages de paramètres, **caractérisé en ce que** l'appareil médical comprend des moyens configurés pour mettre en oeuvre le procédé selon l'une quelconque ou plusieurs des revendications 1 à 12, en vue régler des paramètres relatifs à la sécurité.

14. Appareil médical selon la revendication 13, dans lequel l'appareil médical correspond à un dispositif de traitement sanguin extracorporel.
